(19) Europäisches Patentamt
European Patent Office
Office européen des brevets

(11) **EP 2 250 965 B1**

(12) **EUROPEAN PATENT SPECIFICATION**

(45) Date of publication and mention
of the grant of the patent:
**25.03.2020 Bulletin 2020/13**

(51) Int Cl.:
***A61B 6/00*** *(2006.01)*

(21) Application number: **10161544.1**

(22) Date of filing: **30.04.2010**

(54) **Image acquisition method, device and radiography system**

Bilderfassungsverfahren, Vorrichtung und Röntgenaufnahmesystem

Procédé d'acquisition d'images, dispositif et système de radiographie

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **12.05.2009 CN 200910141210**

(43) Date of publication of application:
**17.11.2010 Bulletin 2010/46**

(73) Proprietor: **GE Medical Systems Global Technology Company LLC
Waukesha,
Wisconsin 53188-1696 (US)**

(72) Inventors:
• **Wang, Dejun**
  **P.R.C. 100176, Beijing (CN)**
• **Li, Huanzhong**
  **P.R.C. 100176, Beijing (CN)**
• **Kadri, Jabri**
  **Waukesha, Wisconsin (US)**
• **Ni, Xianfeng**
  **Waukesha, Wisconsin (US)**
• **Zhang, Tiantian**
  **Waukesha, Wisconsin (US)**

(74) Representative: **Fennell, Gareth Charles et al
Kilburn & Strode LLP
Lacon London
84 Theobalds Road
London WC1X 8NL (GB)**

(56) References cited:
EP-A1- 1 484 016          WO-A1-00/49572
US-A1- 2005 232 397       US-A1- 2008 152 088

**Description**

The present invention generally relates to the field of medical digital radiography systems and, particularly, to an image acquisition method, device and a radiography system.

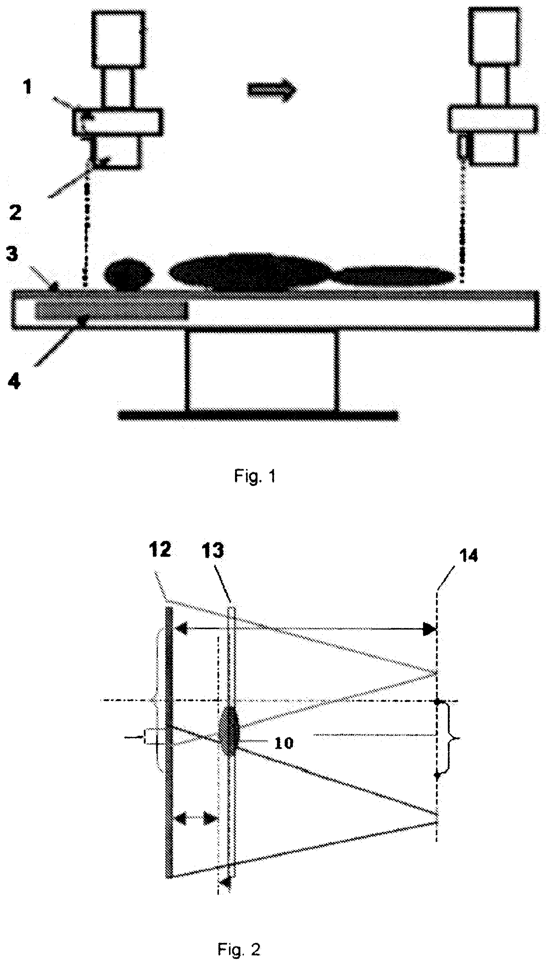

In the medical field, a radiography system is generally used. For example, an X-ray machine is used for imaging a region of interest of a patient, and the doctor then conducts the diagnosis and treatment on the patient according to the obtained images. Figure 1 shows an X-ray machine, and the main parts thereof include an X-ray tube 1, an X-ray collimator 2, a patient securing device 3, a detector 4, wherein the main function of the X-ray tube 1 is to emit X-ray; the main function of the X-ray collimator 2 is to limit the radiation range of light field of X-ray emitted by the X-ray tube 1; the function of the detector 4 lies in receiving X-ray and imaging and then transmitting to a workstation for further processing; the function of the patient securing device 3 lies in two points: the first point is to isolate a patient from the detector 4 for safety, and the second point is to fix a patient so as to minimize the movements of the patient in the whole process of capturing.

As known from said X-ray machine, the tube 1 emits X-ray through a region of interest, which then comes to the X-ray detector 4 so that the image of the region of interest is acquired. The size of the obtained image is generally equal to the size of the X-ray detector. If the field of view of a region of interest is within the size of the X-ray detector, the entire region of interest can be completely presented in one image. For example, the fields of view of regions of interest such as heart, lung and the like are within the size of X-ray detector, so the regions of interest such as heart, lung and the like can be fully shown in an image. Then for some regions of interest, the fields of view of which are larger than the size of the X-ray detector, such as spine, thigh, etc., one image cannot present the entire regions of interest. Such cases need the capturing of a plurality of sub-images, and then pasting these sub-images together to form a complete image that can show the entire region of interest.

EP 1 484 016 discusses acquisition of a composite image with a digital detector.

US 2008/0152088 discusses long length imaging using digital radiography.

At present, for the regions of interest whose fields of view are larger than the X-ray detector size, several methods are used for imaging, falling into two main categories: one category is angulated acquisition method, including capturing multiple sub-images of a region of interest by angulating a tube, i.e. changing the angles of a tube. In other words, capturing a sub-image related to a region of interest when the tube is at a certain angle, and then capturing a sub-image related to the region of interest when the tube is changed to another angle, and so on, till the region of interest is completely covered in all sub-images. Finally, pasting all the sub-images together to form an image of the region of interest. For example, the applicant company's patent US7177455 adopts the method of angulating the tubes to acquire a image of a region of interest.

Said US7177455 mainly has the following defects:

Firstly, due to the need of angulating the tube, a tube angulating positioner is applied. Said tube angulating positioner is very expensive, so the costs of the machines with the use of said method are great.

Secondly, said US7177455 discloses that the first sub-image and the second sub-image have an overlap. As shown in Figure 2, a tube moves on a parallel movement plane 14 of the tube and emits X-ray to irradiate patients. The detector is disposed on a detector incident plane 12. Although the first sub-image and the second sub-image overlap, a region of interest 10 on the plane of the region of interest does not have an overlap, and a part 10 on the region of interest 10 is not included in any sub-image, so the finally acquired image of the region of interest is inaccurate.

The other category is a method of the parallel movement of a tube and an X-ray detector. That is, capturing a sub-image when the tube and the X-ray detector are at a first position, and then simultaneously moving the tube and the X-ray detector in parallel to a second position, and then capturing a sub-image, and so on and so forth, parallelly moving the tube and the X-ray detector in sequence till the end of the region of interest and finally paste the obtained sub-images together to form an image of said region of interest. Such an image mosaic method is to manually move the positions of the tube and the X-ray detector in parallel. That is, after capturing of each sub-image, the operator shall manually move the tube and the X-ray detector in parallel to the next position based on experience. As a result of manual operation, working efficiency is low, and because different operators have different experience, the finally acquired image of a region of interest is often inaccurate.

US6944265 is similar to US7177455. The disclosed overlap thereof is defined on the sub-image plane, namely the first sub-image and the second sub-image overlapping. Thus, US6944265 also has the defects of rendering the finally acquired image of a region of interest inaccurate, similar to US7177455.

One main problem to be addressed by the present invention is to provide an image acquisition method, device and a radiography system to acquire accurate images.

For addressing the above problem, the image acquisition method of various aspects of the present invention as defined in appended claim 1 is used for imaging regions of interest of patients by a radiography system.

Additionally, the value of said overlap is preferably from 5cm to 7cm.

Accordingly, the image acquisition device of various aspects of the present invention as defined in appended claim 3 is used for imaging the regions of interest of patients by a radiography system which comprises a tube and a detector disposed on opposite positions.

Furthermore, the value of said overlap is preferably from 5cm to 7cm.

Another aspect of the present invention provides a radiography system as defined in appended claim 5.

Compared with the prior art, the beneficial effects of the image acquisition method, device and the radiography system of various aspects of the present invention are as follows:

Firstly, the number of the images required to be captured, the positions of the tube and the detector to be moved to and so on are calculated based on the value of the overlap of the region of interest in the adjacent two images, so each of the resulting adjacent images necessarily has an overlap on the plane of the region of interest, guaranteeing the diagnostic effects and the image pasting quality.

Secondly, it is not necessary for the X-ray tube control device in the present invention to have an electric rotation requirement, so costs can be reduced.

Additionally various aspects of the present invention use a mode of determining the starting position and the ending position, and then automatically determining the exposure position, the X-ray field of view, the number of exposures, etc., so these various aspects of the present invention can increase working efficiency and save the operator's time.

For a better understanding of various aspects of the present invention for those skilled in the art, reference is provided to the following detailed description taken in conjunction with the accompanying drawings in which the same reference signs in the drawings refer to the same elements, wherein:

Figure 1 is a schematic drawing of an X-ray machine;

Figure 2 is a schematic drawing of the case of using the method of US7177455 to acquire the image of a region of interest;

Figure 3 is a flowchart of the image acquisition method of various embodiments of this invention;

Figure 4A is a schematic drawing of one example of determining the starting position and the ending position of a region of interest by tube rotation mode;

Figure 4B is a schematic drawing of one example of determining the starting position and the ending position of a region of interest by tube parallel moving mode;

Figure 4C is a schematic drawing of the corresponding relationship between an exposure position and a sub-image obtained by using the technical solution of various aspects of the present invention;

Figure 5 is a flowchart of the calculation step in Figure 3;

Figure 6 is a flowchart of the pasting step in Figure 3;

Figure 7 is a schematic drawing of determining the starting position of a patient's region of interest;

Figure 8 is a schematic drawing of determining the ending position of a patient;

Figure 9 is a schematic drawing of a first exposure position to which a tube and a detector move after calculation;

Figure 10 is a schematic drawing of a second exposure position to which the tube and the detector move to;

Figure 11 is a schematic drawing of a third exposure position to which the tube and the detector move to;

Figure 12 is a schematic drawing of guaranteeing the fixed overlap of the region of interest by using the technical solution of various aspects of the present invention;

Figure 13 shows sub-images acquired by using various embodiments of the present invention and an image obtained by pasting said sub-images;

Figure 14 is a schematic drawing of an image acquisition device of various embodiments of the present invention.

The following describes the features and advantages and so on of various aspects of the present invention by exemplary embodiments.

Once more as shown in Figure 1, said X-ray machine mainly comprises of the X-ray tube 1, the X-ray collimator 2, the patient securing device 3, and the detector 4. The following introduces the technical solution of various aspects of the present invention on the basis of this X-ray machine.

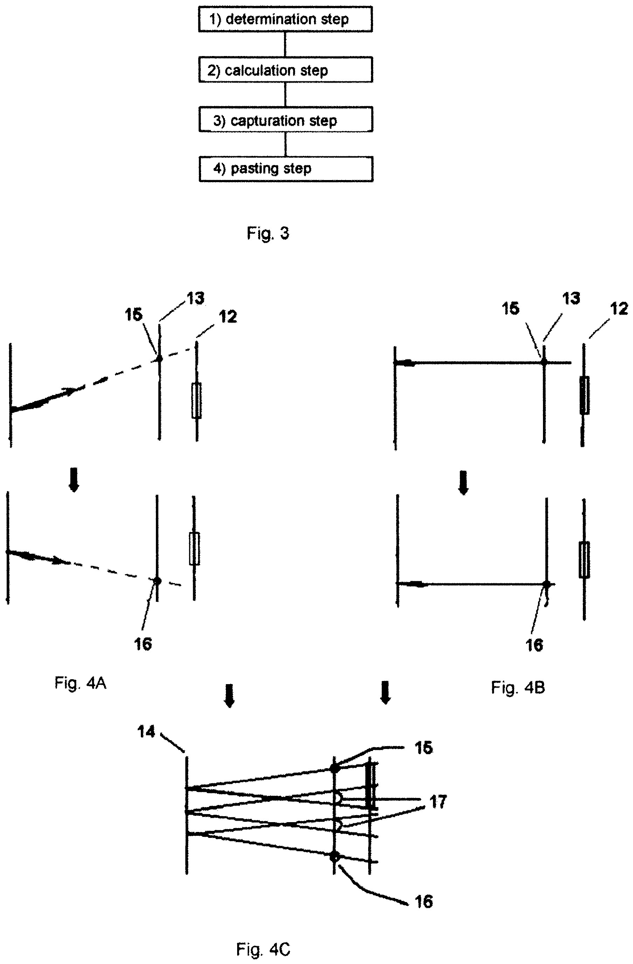

Figure 3 illustrates a flowchart of the image acquisition method of various embodiments of the present invention. The image acquisition method of various embodiments of the present invention is used for imaging regions of interest of patients by an X-ray based machine. Said x-ray machine comprises the tube 1 (see Figure 1) and the detector 4 (see Figure 1) disposed on opposite positions. Said detector 4 is used for receiving X-ray emitted by the tube 1 and generating images. As shown in Figure 3, the image acquisition method comprises: 1) determination step: determining the starting position, the ending position of a region of interest, and the value of overlap of the region of interest in the two adjacent sub-images; 2) calculation step: calculating the number of the sub-images required to be captured, the component of field of view at the direction of tube movement as well as the positions of the tube and the detector corresponding to each sub-image based on the starting position and the ending position of a region of interest and the value of said overlap; 3) capturation step: moving the tube and the detector to each position and capturing the region of interest to obtain several sub-images at said positions; 4) pasting step: pasting the several sub-images together to form an image of the said region of interest.

As known from above, the image acquisition method of various embodiments of the present invention firstly determines the starting position and the ending position of a region of interest, and the value of overlap of the region of interest in the two adjacent sub-images. There can be many modes to determine the starting position and the ending position of a region of interest, such as a mode of angulating a tube or a mode of tube parallel moving, as shown in Figure 4A and Figure 4B, which are schematic drawings of exemplary embodiments to determine the starting position and the ending position of a region of interest. Figure 4A is the angulation determination mode and Figure 4B is the parallel movement determination mode. The angulation determination mode can determine a starting position 15 and an ending position 16 of the region of interest on the plane 13 of the region of interest by rotating the tube 1; the parallel movement determination mode can determine the starting position 15 and the ending position 16 of the region of interest by parallelly moving the tube. Then the number of sub-images required to be captured, the component of the field of view at the direction of tube movement as well as the positions of the tube 1 and the detector 4 corresponding to each sub-image can be calculated based on the starting position 15 and the ending position 16 of the region of interest and the value of said overlap 17. After determining the positions of the tube 1 and the detector 4 for capturing each sub-image, the tube 1 and the detector 4 will be moved to each of the determined positions to capture the region of interest, namely capturing one sub-image in each position, and the number of captured sub-images is equal to the calculated number of sub-images required to be captured. As shown in Figure 4C, the tube 1 moves along a tube parallel moving plane 14, and the detector 4 moves along a detector incident plane 12, and the corresponding relationship between the exposure positions (positions of tube and detector) and the sub-images required to be captured is indicated; finally these captured sub-images are pasted together to obtain the images of regions of interest.

The technical solution of the image acquisition method of various aspects of the present invention can guarantee that there is an overlap of a region of interest in the two adjacent sub-images, rather than as described in US patent US7177455 that just guarantees an overlap of the first sub-image and the second sub-image. An overlap of the first sub-image and the second sub-image does not guarantee the overlap of the region of interest in the sub-images, so the images acquired by using sub-image acquisition method of the present invention are more accurate.

The value of said overlap 17 can be 5cm to 7cm, or also can be other values. Such an overlap value discovered through a series of experiments can achieve the best balance of the number of exposures and the image quality.

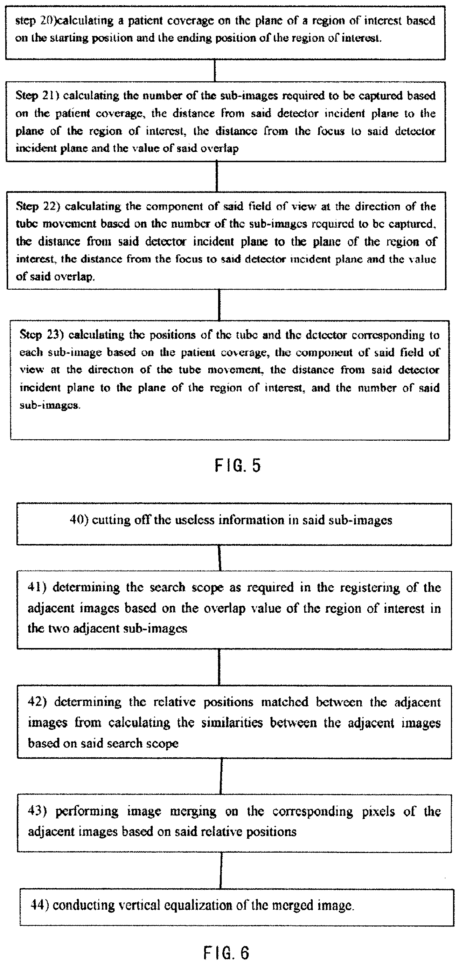

As shown in Figure 5, said calculation step further comprises: Step 20) calculating a patient coverage on the plane of a region of interest based on the starting position and the ending position of the region of interest; Step 21) calculating the number of the sub-images required to be captured based on the patent coverage, the distance from said detector incident plane to the plane of the region of interest, the distance from the focus to said detector incident plane and the value of said overlap; Step 22) calculating the component of the field of view at the direction of the tube movement based on the number of the sub-images required to be captured, the distance from said detector incident plane to the plane of the region of interest, the distance from the focus to said detector incident plane and the value of said overlap; Step 23) calculating the positions of the tube and the detector corresponding to each sub-image based on the patient coverage, the component of said field of view at the direction of the tube movement, the distance from said detector incident plane to the plane of the region of interest, and the number of said sub-images.

Once more as shown in Figure 6, said pasting step 4) further comprises: 40) cutting off the useless information in said sub-images; 41) determining the search scope as required in the registering of the adjacent images based on the overlap value of the region of interest in the two adjacent sub-images; 42) determining the relative positions matched between the adjacent images from calculating the similarities between the adjacent images based on said search scope; 43) performing image merging on the corresponding pixels of the adjacent images based on said relative positions; 44) conducting vertical equalization of the merged image.

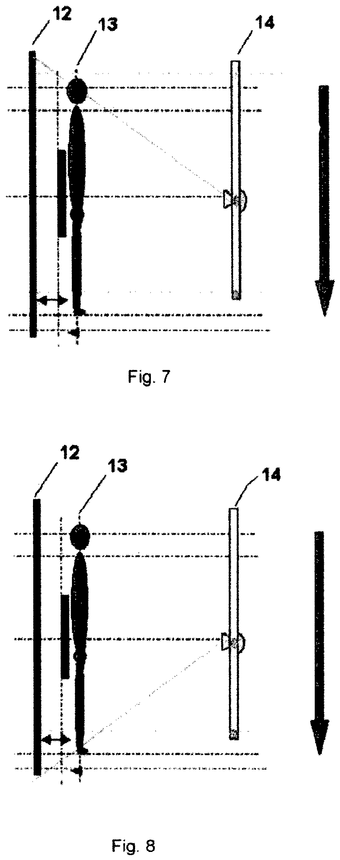

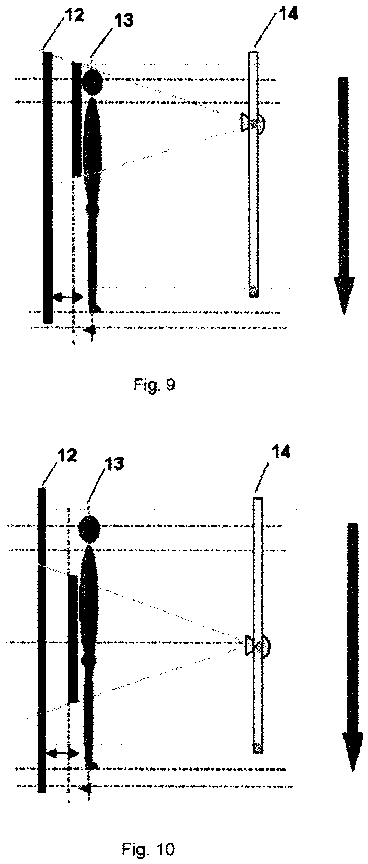

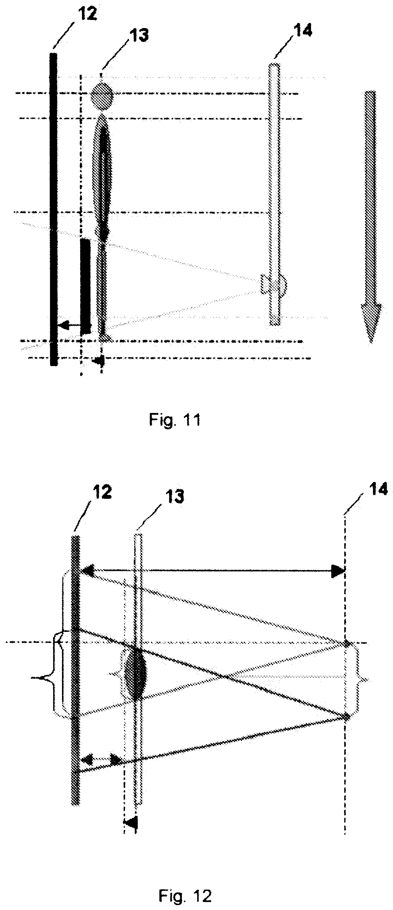

The following describes the technical solution of the image acquisition method of various embodiments of the present invention by an example of acquiring a patient's spine image. As shown in Figures 7-12, the tube moves on the tube parallel moving plane 14, and the detector moves on the detector incident plane 12. Firstly, the starting position topMarkedHt of the region of interest is determined to be 1800mm; the ending position botMarkedHt 1250mm; the overlap value overlap_anat of the region of interest in the two adjacent sub-images is 50mm; suppose the system desires the maximum value of FOV (field of view) Hfov_prefer to be 250mm; Patient coverage on plane 13 of the region of interest is: covAnatPlane=topMarkedHt-botMarkedH=550mm; Provisional moving distance of the X-ray tube and the detector each time is:

$$\text{DFS\_tmp} = \text{Hfov\_prefer} - \text{Hfov\_prefer} * (\text{detAnatSep}/\text{acqSID}) - \text{overlap\_anat},$$

wherein detAnatSep indicates the distance from the detector incident plane 12 to a plane 13 of the region of interest (constant), acqSID indicates the vertical distance from the tube focus to the detector incident plane 12 (constant); The number of tube and detector movements is:

$$N = \text{ceil}((\text{covAnatPlane} - \text{Hfov\_prefer} * (\text{acqSID} - \text{detAnatSep})/\text{acqSID})/\text{DFS\_tmp}),$$

wherein the function ceil() indicates that real number is rounded up to an integer;

The final component of field of view at the direction of tube movement is:

$$\text{VertColl} = (\text{covAnatPlane} + \text{overlap\_anat}*N)/(N+(\text{acqSID}-\text{detAnatSep})/\text{acqSID} - N*\text{detAnatSep}/\text{acqSID});$$

The final movement distance of the tube and the detector is:

$$\text{DFS} = (\text{covAnatPlane} - \text{VertColl} * (\text{acqSID} - \text{detAnatSep})/\text{acqSID})/N;$$

The overlap value on the detector incident plane 12 is:

$$\text{overlap} = \text{VertColl} - \text{DFS};$$

The final number of exposures is N+1;

The position of the tube and the detector corresponding to each sub-image:
i from 1 to N+1;

$$\text{location}(i) = \text{topMarkedHt} - \text{DFS} * (i-1) - (1/2) * ((\text{acqSID}-\text{COI}-\text{detBarrierSep})/\text{acqSID}) * \text{VertColl};$$

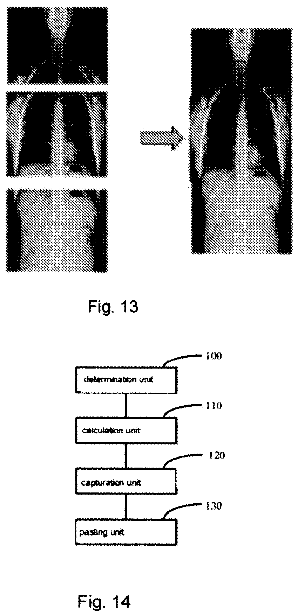

For the present example, because the starting position topMarkedHt of the region of interest is 1800mm; the ending position botMarkedHt is 1250mm; the overlap value of the region of interest in the two adjacent sub-images overlap_anat is 50mm; suppose the system desires a component of field of view at the direction of tube movement to be 250mm; through the above calculation formula, we firstly obtain the number of the tube and the detector movements is 2, and then the final component of field of view at the direction of tube movement is 243.75mm; afterwards the obtained final movement distance of the tube and the detector is 166.67mm; finally the obtained final number of exposures is 3, and the positions of the tube and the detector in each exposure are respectively 1691.67mm, 1525mm, 1358.33mm. Then, moving the tube and the detector to 1691.67mm, 1525mm, 1358.33mm to capture, and as shown in Figure 13, the left shows the captured three sub-images.

After obtaining the three sub-images, cutting off the useless information in these sub-images, e.g. the sub-images beyond limitation scope of the collimator 2 (see Figure 1); determining the search scope as required in registering of the adjacent images based on the overlap value on said detector incident plane, which is about 70mm; the following is to calculate the similarities between the adjacent images based on the search scope so as to determine the relative positions matched between the adjacent images; performing image merging on the corresponding pixels of the adjacent images based on said relative positions; then conducting vertical equalization of the merged image so as to paste the three sub-images into one image, as shown in the right of Figure 13, wherein said vertical equalization means standardizing each sub-image so that the brightness and contrast and so on of each sub-image are equalized.

To obtain the more accurate images, further processing can be conducted to the images, such as image enhancement methods like tissue equalization, multi-resolution processing, contrast stretching.

Other modes can also be applied for the pasting step, such as methods of mAs SCALING, BLENDING and so on.

For the moving direction of the tube 1, it can be horizontal moving, vertical moving or moving at a certain angle.

As shown in Figure 14, the image acquisition device of the present invention comprises: determination unit 100: or determining the starting position, the ending position of a region of interest, and the value of overlap of the region of interest in the two adjacent sub-images; calculation unit 110: for calculating the number of the sub-images required to be captured, the component of field of view at the direction of tube movement as well as the positions of the tube and the detector corresponding to each sub-image based on the starting position and the ending position of a region of interest and the value of said overlap; capturation unit 120: for moving the tube and the detector to each of the positions and controlling the tube to capture the region of interest to obtain several sub-images at said positions; pasting unit 130: for pasting the several sub-images together to form an image of the said region of interest.

Wherein said calculation unit 110 further comprises: the first unit, for calculating a patient coverage on the plane of a region of interest based on the starting position and the ending position of the region of interest; the second unit, for calculating the number of the sub-images required to be captured based on the patent coverage, the distance from said detector incident plane to the plane of the region of interest, the distance from the focus to said detector incident plane and the value of said overlap; the third unit, for calculating the component of said field of view at the direction of the tube movement based on the number of the sub-images required to be captured, the distance from said detector incident plane to the plane of the region of interest, the distance from the focus to said detector incident plane and the value of said overlap; the fourth unit, for calculating the positions of the tube and the detector corresponding to each sub-image based on the patient coverage, the component of said field of view at the direction of the tube movement, the distance from said detector incident plane to the plane of the region of interest, and the number of said sub-images.

Additionally, said pasting unit 130 further comprises: cutting unit, for cutting off the useless information in said sub-images; search scope determining unit, for determining the search scope as required in the registering of the adjacent images based on the overlap value of the region of interest in the two adjacent sub-images; relative position determining unit, for determining the relative positions matched between the adjacent images from calculating the similarities between the adjacent images based on said search scope; merging unit, for performing image merging on the corresponding pixels of the adjacent images based on said relative positions; vertical equalization unit, for conducting vertical equalization of the merged image. The value of said overlap can be 5cm to 7cm or other values, preferably 5cm.

Embodiments of the present invention also provide a radiography system. Said radiography system comprises a tube and a detector disposed on opposite positions, wherein said radiography system further comprises an image acquisition device. The image acquisition device comprises: determination unit 100: for determining the starting position, the ending position of a region of interest, and the value of overlap of the region of interest in the two adjacent sub-images; calculation unit 110: for calculating the number of the sub-images required to be captured, the component of field of view at the direction of tube movement as well as the positions of the tube and the detector corresponding to each sub-image based on the starting position and the ending position of a region of interest and the value of said overlap; capturation unit 120: moving the tube and the detector to each position and controlling the tube to capture the region of interest to obtain several sub-images at said positions; pasting unit 130: pasting the several sub-images together to form an image of the said region of interest.

Wherein said calculation unit 110 further comprises: the first unit, for calculating a patient coverage on the plane of a region of interest based on the starting position and the ending position of the region of interest; the second unit, for calculating the number of the sub-images required to be captured based on the patent coverage, the distance from said detector incident plane to the plane of the region of interest, the distance from the focus to said detector incident plane and the value of said overlap; the third unit, for calculating the component of said field of view at the direction of the tube movement based on the number of the sub-images required to be captured, the distance from said detector incident plane to the plane of the region of interest, the distance from the focus to said detector incident plane and the value of said overlap; the fourth unit, for calculating the positions of the tube and the detector corresponding to each sub-image based on the patient coverage, the component of said field of view at the direction of the tube movement, the distance from said detector incident plane to the plane of the region of interest, and the number of said sub-images.

Additionally, said pasting unit 130 further comprises: cutting unit, for cutting off the useless information in said sub-images; search scope determining unit, for determining the search scope as required in the registering of the adjacent images based on the overlap value of the region of interest in the two adjacent sub-images; relative position determining unit, for determining the relative positions matched between the adjacent images from calculating the similarities between the adjacent images based on said search scope; merging unit, for performing image merging on the corresponding pixels of the adjacent images based on said relative positions; vertical equalization unit, for conducting vertical equalization of the merged image.

To sum up, firstly, the number of the images required to be captured, the positions of the tube and the detector to be moved to and so on are calculated based on the value of the overlap of the region of interest in the adjacent two images, so each of the resulting adjacent images necessarily has an overlap on the plane of the region of interest, guaranteeing the diagnostic effects and the image pasting quality; Secondly, it is not necessary for the X-ray tube control device to have an electric rotation requirement, so costs can be reduced; Finally, various aspects of the present invention use a mode of determining the starting position and the ending position, and then automatically determining the exposure position, the X-ray field of view, the number of exposures, etc., so aspects of the present invention can increase working efficiency and save the operator's time.

Various features of the invention have been described with reference to various specific examples. However, it should be understood that many variations and modifications may be made by those skilled in the art without departing from the scope of the invention. All such modifications and changes are intended to be included in the scope that is defined by the accompanying claims.

## Claims

1. An image acquisition method for imaging regions of interest (10) of patients by a radiography system, wherein said radiography system comprises an X-ray tube (1) that moves on a tube parallel moving plane (14) and a detector (4) disposed opposite the X-ray tube (1), said image acquisition method comprises:

   determination step: determining the starting position (15), the ending position (16) of a region of interest (10), and the value of overlap (overlap_anat) of the region of interest in two adjacent sub-images;
   calculation step: calculating the number of the sub-images required to be captured, the component of field of view, of the X-ray tube (1), at the direction of tube movement as well as the positions of the X-ray tube (1) and the detector (4) corresponding to each sub-image based on the starting position and the ending position of the region of interest and the value of said overlap, said plane (13) being disposed at a distance (detAnatSep) from a detector incident plane (12);
   capturation step: moving the tube along a tube parallel moving plane (14) and the detector along a detector incident plane (12) in a parallel movement determination mode to each position and capturing the region of interest to obtain several sub-images at said positions, said X-ray tube (1) and said detector (4) being moved a distance of

$$DFS = (covAnatPlane - VertColl * (acqSID - detAnatSep)/acqSID)/N,$$

   where covAnat plane is the patient coverage on the plane (13) of the region of interest, where

$$VertColl = (covAnatPlane + overlap\_anat*N) / (N+(acqSID-detAnatSep) / acqSID-N*detAnatSep/acqSID),$$

   where acqSID indicates a vertical distance from a tube focus to the detector incident plane (12), where overlap_anat is the value of said overlap,
   where N = ceil ((covAnatPlane - Hfov_prefer*(acqSID - detAnatSep) / acqSID) / DFS tmp),
   where Hfov_prefer is a maximum field of view, where

$$DFS\_tmp=Hfov\_prefer-Hfov\_prefer*(detAnatSep/acqSID)-overlap\_anat,$$

   pasting step: pasting the several sub-images together to form an image of the said region of interest;

   wherein said calculation step comprises:

calculating a patient coverage on the plane of a region of interest based on the starting position and the ending position of the region of interest;

calculating the number of the sub-images required to be captured based on the patient coverage, the distance from said detector incident plane to the plane of the region of interest, the distance from the focus to said detector incident plane and the value of said overlap;

calculating the component of said field of view at the direction of the X-ray tube (1) movement based on the number of the sub-images required to be captured, the distance from said detector incident plane to the plane of the region of interest, the distance from the focus to said detector incident plane and the value of said overlap;

calculating the positions of the tube and the detector corresponding to each sub-image based on the patient coverage, the component of said field of view at the direction of the tube movement, the distance from said detector incident plane to the plane of the region of interest, and the number of said sub-images;

wherein said pasting step comprises:

cutting off the useless information in said sub-images;

determining the search scope as required in the registering of the adjacent images based on the overlap value of the region of interest in the two adjacent sub-images;

determining the relative positions matched between the adjacent images from calculating the similarities between the adjacent images based on said search scope;

performing image merging on the corresponding pixels of the adjacent images based on said relative positions;

conducting vertical equalization of the merged image, wherein vertical equalization comprises standardizing each sub-image so that brightness and contrast are equalized.

2. The image acquisition method according to claim 1, **characterized in that** the value of said overlap is preferably from 5cm to 7cm.

3. An image acquisition device for imaging regions of interest of patients by radiography system, wherein said radiography system comprises an X-ray tube (1) that moves on a tube moving parallel moving plane (14) and a detector (4) disposed opposite the X-ray tube (1), wherein said image acquisition device comprises:

determination unit (100), for determining the starting position, the ending position of a region of interest, and the value of overlap (overlap_anat) of the region of interest in two adjacent sub-images;

calculation unit (110), for calculating the number of the sub-images required to be obtained, the component of field of view, of the X-ray tube (1), at the direction of tube movement as well as the positions of the X-ray tube (1) and the detector (4) corresponding to each sub-image based on the starting position and the ending position of the region of interest and the value of said overlap, said plane (13) being disposed at a distance (detAnatSep) from a detector incident plane (12);

capturation unit (120), for moving the tube along a tube parallel moving plane (14) and the detector along a detector incident plane (12) in a parallel movement determination mode to each position and capturing the region of interest to obtain several sub-images at said positions, said X-ray tube (1) and said detector (4) being moved a distance of

$$DFS = (covAnatPlane - VertColl * (acqSID - detAnatSep)/acqSID)/N,$$

where covAnat plane is the patient coverage on the plane (13) of the region of interest, where

$$VertColl = (covAnatPlane + overlap\_anat*N) / (N+(acqSID-detAnatSep) / acqSID-N*detAnatSep/acqSID),$$

where acqSID indicates a vertical distance from a tube focus to the detector incident plane (12), where overlap_anat is the value of said overlap, where

$$N = \mathrm{ceil}\ ((\mathrm{covAnatPlane} - \mathrm{Hfov\_prefer} * (\mathrm{acqSID} - \mathrm{detAnatSep}) / \mathrm{acqSID}) / \mathrm{DFS\_tmp}),$$

where Hfov_prefer is a maximum field of view, where

$$\mathrm{DFS\_tmp} = \mathrm{Hfov\_prefer} - \mathrm{Hfov\_prefer} * (\mathrm{detAnatSep}/\mathrm{acqSID}) - \mathrm{overlap\_anat},$$

pasting unit (130), for pasting the several sub-images together to form an image of the said region of interest;

wherein said calculation unit (110) comprises:

a first unit, for calculating a patient coverage on the plane (13) of a region of interest based on the starting position and the ending position of the region of interest;
a second unit, for calculating the number of the sub-images required to be captured based on the patient coverage, the distance from said detector incident plane (12) to the plane of the region of interest, the distance from the focus to said detector incident plane and the value of said overlap;
a third unit, for calculating the component of said field of view at the direction of the tube movement based on the number of the sub-images required to be captured, the distance from said detector incident plane (12) to the plane (13) of the region of interest, the distance from the focus to said detector incident plane and the value of said overlap;
a fourth unit, for calculating the positions of the tube and the detector corresponding to each sub-image based on the patient coverage, the component of said field of view at the direction of the X-ray tube (1) movement, the distance from said detector incident plane (12) to the plane (13) of the region of interest, and the number of said sub-images;

wherein said pasting unit (130) comprises:

cutting unit, for cutting off redundant information in said sub-images;
search scope determining unit, for determining the search scope as required in the registering of the adjacent images based on the overlap value of the region of interest in the two adjacent sub-images;
relative position determining unit, used for determining the relative positions matched between the adjacent images from calculating the similarities between the adjacent images based on said search scope;
merging unit, for performing image merging on the corresponding pixels of the adjacent images based on said relative positions;
vertical equalization unit, for conducting vertical equalization of the merged image, wherein vertical equalization comprises standardizing each sub-image so that brightness and contrast are equalized.

4. The image acquisition device according to claim 3, **characterized in that** the value of said overlap is preferably from 5cm to 7cm.

5. A radiography system, said radiography system comprising an X-ray tube (1) and a detector (4) disposed on opposite positions, **characterized in that** said radiography system further comprises an image acquisition device as recited in claims 3 or 4.

**Patentansprüche**

1. Bilderfassungsverfahren zur Darstellung von interessierenden Bereichen (10) von Patienten durch ein Röntgenaufnahmesystem, wobei das Röntgenaufnahmesystem eine Röntgenröhre (1), die sich auf einer parallelen Bewegungsebene (14) der Röhre bewegt, und einen gegenüber der Röntgenröhre (1) angeordneten Detektor (4) umfasst, wobei das Bilderfassungsverfahren das Folgende umfasst:

Bestimmungsschritt: Bestimmen der Startposition (15), der Endposition (16) eines interessierenden Bereichs (10) und des Überlappungswerts (overlap_anat) des interessierenden Bereichs in zwei benachbarten Teilbildern;

Berechnungsschritt: Berechnen der Anzahl der Teilbilder, die erfasst werden müssen, der Komponente des Sichtfelds, der Röntgenröhre (1), in der Richtung der Röhrenbewegung, sowie der Positionen der Röntgenröhre (1) und des Detektors (4), die jedem Teilbild entsprechen, auf Basis der Startposition und der Endposition des interessierenden Bereichs und des Werts der Überlappung, wobei die Ebene (13) in einem Abstand (detAnatSep) von einer Einfallsebene (12) des Detektors angeordnet ist;

Erfassungsschritt: Bewegen der Röhre entlang einer parallelen Bewegungsebene (14) der Röhre und des Detektors entlang einer Einfallsebene (12) des Detektors in einem parallelen Bewegungsbestimmungsmodus zu jeder Position und Erfassen des interessierenden Bereichs, um mehrere Teilbilder an diesen Positionen zu erhalten, wobei die Röntgenröhre (1) und der Detektor (4) über einen Abstand bewegt werden

$$DFS = (covAnatPlane - VertColl * (acqSID - detAnatSep)/acqSID)/N,$$

wobei covAnatPlane die Patientenabdeckung auf der Ebene (13) des interessierenden Bereichs ist, wobei

$$VertColl = (covAnatPlane + overlap\_anat*N) / (N+(acqSID-detAnatSep) / acqSID-N*detAnatSep/acqSID),$$

wobei acqSID auf einen vertikalen Abstand von einem Röhrenfokus zur Einfallsebene (12) des Detektors hinweist, wobei overlap_anat der Wert der Überlappung ist, wobei

$$N = ceil ((covAnatPlane - Hfov\_prefer * (acqSID - detAnatSep) / acqSID) / DFS\_tmp),$$

wobei Hfov_prefer ein maximales Sichtfeld ist, wobei

$$DFS\_tmp=Hfov\_prefer- Hfov\_prefer*(detAnatSep/acqSID)-overlap\_anat,$$

Übertragungsschritt: gemeinsames Übertragen der mehreren Teilbilder zur Bildung eines Bilds des interessierenden Bereichs;

wobei der Berechnungsschritt das Folgende umfasst:

Berechnen einer Patientenabdeckung auf der Ebene eines interessierenden Bereichs auf Basis der Startposition und der Endposition des interessierenden Bereichs;

Berechnen der Anzahl der Teilbilder, die erfasst werden müssen, auf Basis der Patientenabdeckung, des Abstands von der Einfallsebene des Detektors zur Ebene des interessierenden Bereichs, des Abstands vom Fokus zur Einfallsebene des Detektors und des Werts der Überlappung;

Berechnen der Komponente des Sichtfelds in der Richtung der Bewegung der Röntgenröhre (1) auf Basis der Anzahl der Teilbilder, die erfasst werden müssen, des Abstands von der Einfallsebene des Detektors zur Ebene des interessierenden Bereichs, des Abstands vom Fokus zur Einfallsebene des Detektors und des Werts der Überlappung;

Berechnen der Positionen der Röhre und des Detektors, die jedem Teilbild entsprechen, auf Basis der Patientenabdeckung, der Komponente des Sichtfelds in der Richtung der Röhrenbewegung, des Abstands von der Einfallsebene des Detektors zur Ebene des interessierenden Bereichs und der Anzahl der Teilbilder;

wobei der Übertragungsschritt das Folgende umfasst:

Ausschneiden der nutzlosen Informationen in den Teilbildern;

Bestimmen des in der Registrierung der benachbarten Bilder erforderlichen Suchumfangs auf Basis des Überlappungswerts des interessierenden Bereichs in den zwei benachbarten Teilbildern;

Bestimmen der zwischen den benachbarten Bildern angepassten relativen Positionen aus der Berechnung der Ähnlichkeiten zwischen den benachbarten Bildern auf Basis des Suchumfangs;

Durchführen einer Bildfusion an den entsprechenden Pixeln der benachbarten Bilder auf Basis der relativen Positionen;

Durchführen vertikaler Egalisierung des zusammengeführten Bilds, wobei vertikale Egalisierung die Standardisierung jedes Teilbilds umfasst, so dass Helligkeit und Kontrast ausgeglichen werden.

2. Bilderfassungsverfahren nach Anspruch 1, **dadurch gekennzeichnet, dass** der Wert der Überlappung vorzugsweise 5 cm bis 7 cm beträgt.

3. Bilderfassungsvorrichtung zur Darstellung von interessierenden Bereichen von Patienten durch ein Röntgenaufnahmesystem, wobei das Röntgenaufnahmesystem eine Röntgenröhre (1), die sich auf einer parallelen Bewegungsebene (14) der Röhre bewegt, und einen gegenüber der Röntgenröhre (1) angeordneten Detektor (4) umfasst, wobei die Bilderfassungsvorrichtung das Folgende umfasst:

eine Bestimmungseinheit (100) zum Bestimmen der Startposition, der Endposition eines interessierenden Bereichs und des Überlappungswerts (overlap_anat) des interessierenden Bereichs in zwei benachbarten Teilbildern;

eine Berechnungseinheit (110) zum Berechnen der Anzahl der Teilbilder, die erfasst werden müssen, der Komponente des Sichtfelds, der Röntgenröhre (1), in der Richtung der Röhrenbewegung, sowie der Positionen der Röntgenröhre (1) und des Detektors (4), die jedem Teilbild entsprechen, auf Basis der Startposition und der Endposition des interessierenden Bereichs und des Werts der Überlappung, wobei die Ebene (13) in einem Abstand (detAnatSep) von einer Einfallsebene (12) des Detektors angeordnet ist;

eine Erfassungseinheit (120) zum Bewegen der Röhre entlang einer parallelen Bewegungsebene (14) der Röhre und des Detektors entlang einer Einfallsebene (12) des Detektors in einem parallelen Bewegungsbestimmungsmodus zu jeder Position und Erfassen des interessierenden Bereichs, um mehrere Teilbilder an diesen Positionen zu erhalten, wobei die Röntgenröhre (1) und der Detektor (4) über einen Abstand bewegt werden

```
DFS   =   (covAnatPlane   -   VertColl   *   (acqSID   -
detAnatSep)/acqSID)/N,
```

wobei covAnatPlane die Patientenabdeckung auf der Ebene (13) des interessierenden Bereichs ist, wobei

```
VertColl  =  (covAnatPlane  +  overlap_anat*N)  /
(N+(acqSID-detAnatSep) / acqSID-N*detAnatSep/acqSID),
```

wobei acqSID auf einen vertikalen Abstand von einem Röhrenfokus zur Einfallsebene (12) des Detektors hinweist, wobei overlap_anat der Wert der Überlappung ist, wobei

```
N = ceil ((covAnatPlane - Hfov_prefer * (acqSID -
detAnatSep) / acqSID) / DFS_tmp),
```

wobei Hfov_prefer ein maximales Sichtfeld ist, wobei

```
DFS_tmp=Hfov_prefer-
Hfov_prefer*(detAnatSep/acqSID)-overlap_anat,
```

eine Übertragungseinheit (130) zum gemeinsamen Übertragen der mehreren Teilbilder zur Bildung eines Bilds des interessierenden Bereichs;

wobei die Berechnungseinheit (110) das Folgende umfasst:

eine erste Einheit zum Berechnen einer Patientenabdeckung auf der Ebene (13) eines interessierenden Bereichs auf Basis der Startposition und der Endposition des interessierenden Bereichs;

eine zweite Einheit zum Berechnen der Anzahl der Teilbilder, die erfasst werden müssen, auf Basis der Patientenabdeckung, des Abstands von der Einfallsebene (12) des Detektors zur Ebene des interessierenden Bereichs, des Abstands vom Fokus zur Einfallsebene des Detektors und des Werts der Überlappung;

eine dritte Einheit zum Berechnen der Komponente des Sichtfelds in der Richtung der Röhrenbewegung auf Basis der Anzahl der Teilbilder, die erfasst werden müssen, des Abstands von der Einfallsebene (12) des Detektors zur Ebene (13) des interessierenden Bereichs, des Abstands vom Fokus zur Einfallsebene des Detektors und des Werts der Überlappung;

eine vierte Einheit zum Berechnen der Positionen der Röhre und des Detektors, die jedem Teilbild entsprechen, auf Basis der Patientenabdeckung, der Komponente des Sichtfelds in der Richtung der Bewegung der Röntgenröhre (1), des Abstands von der Einfallsebene (12) des Detektors zur Ebene (13) des interessierenden Bereichs und der Anzahl der Teilbilder;

wobei die Übertragungseinheit (130) das Folgende umfasst:

eine Schneideeinheit zum Ausschneiden überflüssiger Informationen in den Teilbildern;

eine Suchumfangsbestimmungseinheit zum Bestimmen des in der Registrierung der benachbarten Bilder erforderlichen Suchumfangs auf Basis des Überlappungswerts des interessierenden Bereichs in den zwei benachbarten Teilbildern;

eine Bestimmungseinheit für die relativen Positionen zum Bestimmen der zwischen den benachbarten Bildern angepassten relativen Positionen aus der Berechnung der Ähnlichkeiten zwischen den benachbarten Bildern auf Basis des Suchumfangs;

Fusionseinheit zum Durchführen einer Bildfusion an den entsprechenden Pixeln der benachbarten Bilder auf Basis der relativen Positionen;

eine vertikale Egalisierungseinheit zum Durchführen vertikaler Egalisierung des zusammengeführten Bilds, wobei vertikale Egalisierung die Standardisierung jedes Teilbilds umfasst, so dass Helligkeit und Kontrast ausgeglichen werden.

4. Bilderfassungsvorrichtung nach Anspruch 3, **dadurch gekennzeichnet, dass** der Wert der Überlappung vorzugsweise 5 cm bis 7 cm beträgt.

5. Röntgenaufnahmesystem, wobei das Röntgenaufnahmesystem eine Röntgenröhre (1) und einen Detektor (4) umfasst, die auf gegenüberliegenden Positionen angeordnet sind, **dadurch gekennzeichnet, dass** das Röntgenaufnahmesystem ferner eine Bilderfassungsvorrichtung nach den Ansprüchen 3 oder 4 umfasst.

**Revendications**

1. Procédé d'acquisition d'image pour l'imagerie de régions d'intérêt (10) de patients par un système radiographique, dans lequel ledit système radiographique comprend un tube à rayons X (1) qui se déplace sur un plan mobile (14) parallèle au tube et un détecteur (4) disposé en regard du tube à rayons X (1), ledit procédé d'acquisition d'image comprenant :

une étape de détermination consistant à : déterminer la position de début (15), la position de fin (16) d'une région d'intérêt (10), et la valeur de chevauchement (overlap_anat) de la région d'intérêt dans deux sous-images adjacentes ;

une étape de calcul consistant à : calculer le nombre des sous-images devant être capturées, la composante du champ de vision, du tube à rayons X (1), dans la direction du mouvement du tube ainsi que les positions du tube à rayons X (1) et du détecteur (4) correspondant à chaque sous-image en se basant sur la position de début et la position de fin de la région d'intérêt et la valeur dudit chevauchement, ledit plan (13) étant disposé à une certaine distance (detAnatSep) d'un plan (12) incident au détecteur ;

une étape de capture consistant à : déplacer le tube le long d'un plan mobile (14) parallèle au tube et le détecteur le long d'un plan (12) incident au détecteur dans un mode de détermination de mouvement parallèle vers chaque position et capturer la région d'intérêt pour obtenir plusieurs sous-images au niveau desdites sous-positions, ledit tube à rayons X (1) et ledit détecteur (4) étant déplacés sur une distance de

$$DFS = (covAnatPlane - VertColl * (acqSID - detAnatSep)/acqSID)/N,$$

où le plan covAnat est la couverture du patient sur le plan (13) de la région d'intérêt, où

$$VertColl = (covAnatPlane + overlap\_anat*N) / (N+(acqSID-detAnatSep) / acqSID-N*detAnatSep/acqSID),$$

où acqSID désigne une distance verticale à partir d'un foyer du tube vers le plan (12) incident au détecteur, où overlap_anat est la valeur dudit chevauchement où

$$N = ceil ((covAnatPlane - Hfov\_prefer * (acqSID - detAnatSep) / acqSID) / DFS\_tmp),$$

où Hfov_prefer est un champ de vision maximum, où

$$DFS\_tmp = Hfov\_prefer - Hfov\_prefer*(detAnatSep/acqSID) - overlap\_anat,$$

une étape de collage consistant à : coller ensemble les multiples sous-images pour former une image de ladite région d'intérêt ;

dans lequel ladite étape de calcul consiste à :

calculer une couverture de patient sur le plan d'une région d'intérêt en se basant sur la position de début et la position de fin de la région d'intérêt ;
calculer le nombre des sous-images devant être capturées en se basant sur la couverture du patient, la distance depuis le plan incident audit détecteur vers le plan de la région d'intérêt, la distance depuis le foyer vers ledit plan incident au détecteur et la valeur dudit chevauchement ;
calculer la composante dudit champ de vision dans la direction du mouvement du tube à rayons X (1) en se basant sur le nombre des sous-images devant être capturées, la distance depuis ledit plan incident au détecteur vers le plan de la région d'intérêt, la distance depuis le foyer vers ledit plan incident au détecteur et la valeur dudit chevauchement ;
calculer les positions du tube et du détecteur correspondant à chaque sous-image en se basant sur la couverture du patient, la composante dudit champ de vision dans la direction du mouvement du tube, la distance depuis ledit plan incident au détecteur vers le plan de la région d'intérêt, et le nombre desdites sous-images ;

dans lequel ladite étape de collage consiste à :

éliminer par découpage les informations inutiles dans lesdites sous-images ;
déterminer la portée de recherche requise dans l'enregistrement des images adjacentes en se basant sur la valeur de chevauchement de la région d'intérêt dans les deux sous-images adjacentes ;
déterminer les positions relatives concordantes entre les images adjacentes à partir du calcul des similitudes entre les images adjacentes en se basant sur ladite portée de recherche ;
réaliser une fusion d'images sur les pixels correspondants des images adjacentes en se basant sur lesdites positions relatives ;
effectuer une égalisation verticale de l'image fusionnée, l'égalisation verticale consistant à normaliser chaque sous-image de sorte que la luminosité et le contraste soient égalisés.

**2.** Procédé d'acquisition d'image selon la revendication 1, **caractérisé en ce que** la valeur dudit chevauchement est de préférence de 5 cm à 7 cm.

**3.** Dispositif d'acquisition d'image pour l'imagerie de régions d'intérêt de patients par un système radiographique, dans lequel ledit système radiographique comprend un tube à rayons X (1) qui se déplace sur un plan mobile (14) parallèle au tube et un détecteur (4) disposé en regard du tube à rayons X (1), ledit dispositif d'acquisition d'image comprenant :

une unité de détermination (100), pour déterminer la position de début, la position de fin d'une région d'intérêt, et la valeur de chevauchement (overlap_anat) de la région d'intérêt dans deux sous-images adjacentes ;
une unité de calcul (110), pour calculer le nombre des sous-images devant être obtenues, la composante du champ de vision, du tube à rayons X (1), dans la direction du mouvement du tube ainsi que les positions du tube à rayons X (1) et du détecteur (4) correspondant à chaque sous-image en se basant sur la position de début et la position de fin de la région d'intérêt et la valeur dudit chevauchement, ledit plan (13) étant disposé à une certaine distance (detAnatSep) d'un plan (12) incident au détecteur ;
une unité de capture (120), pour déplacer le tube le long d'un plan mobile (14) parallèle au tube et le détecteur le long d'un plan (12) incident au détecteur dans un mode de détermination de mouvement parallèle vers chaque position et capturer la région d'intérêt pour obtenir plusieurs sous-images au niveau desdites positions, ledit tube à rayons X (1) et ledit détecteur (4) étant déplacés sur une distance de

$$DFS = (covAnatPlane - VertColl * (acqSID - detAnatSep)/acqSID)/N,$$

où le plan covAnat est la couverture du patient sur le plan (13) de la région d'intérêt, où

$$VertColl = (covAnatPlane + overlap\_anat*N) / (N+(acqSID-detAnatSep) / acqSID-N*detAnatSep/acqSID),$$

où acqSID désigne une distance verticale à partir d'un foyer du tube vers le plan (12) incident au détecteur, où overlap_anat est la valeur dudit chevauchement, où

$$N = ceil ((covAnatPlane - Hfov\_prefer * (acqSID - detAnatSep) / acqSID) / DFS\_tmp),$$

où Hfov_prefer est un champ de vision maximum, où

$$DFS\_tmp = Hfov\_prefer - Hfov\_prefer*(detAnatSep/acqSID) - overlap\_anat,$$

une unité de collage (130), pour coller ensemble les multiples sous-images pour former une image de ladite région d'intérêt ;

dans lequel ladite unité de calcul (110) comprend :

une première unité, pour calculer une couverture de patient sur le plan (13) d'une région d'intérêt en se basant sur la position de début et la position de fin de la région d'intérêt ;
une deuxième unité, pour calculer le nombre des sous-images devant être capturées en se basant sur la couverture du patient, la distance depuis le plan (12) incident audit détecteur vers le plan de la région d'intérêt, la distance depuis le foyer vers ledit plan incident au détecteur et la valeur dudit chevauchement ;
une troisième unité, pour calculer la composante dudit champ de vision dans la direction du mouvement du tube en se basant sur le nombre des sous-images devant être capturées, la distance depuis ledit plan (12) incident au détecteur vers le plan (13) de la région d'intérêt, la distance depuis le foyer vers ledit plan incident au détecteur et la valeur dudit chevauchement ;
une quatrième unité, pour calculer les positions du tube et du détecteur correspondant à chaque sous-image en se basant sur la couverture du patient, la composante dudit champ de vision dans la direction du mouvement du tube à rayons X (1), la distance depuis ledit plan (12) incident au détecteur vers le plan (13) de la région d'intérêt, et le nombre desdites sous-images ;

dans lequel ladite unité de collage (130) comprend :

une unité de découpe, pour éliminer par découpage les informations inutiles dans lesdites sous-images ;
une unité de détermination de portée de recherche, pour déterminer la portée de recherche requise dans l'enregistrement des images adjacentes en se basant sur la valeur de chevauchement de la région d'intérêt dans les deux sous-images adjacentes ;
une unité de détermination de positions relatives, servant à déterminer les positions relatives concordantes entre les images adjacentes à partir du calcul des similitudes entre les images adjacentes en se basant sur ladite portée de recherche ;
une unité de fusion, pour réaliser une fusion d'images sur les pixels correspondants des images adjacentes en se basant sur lesdites positions relatives ;
une unité d'égalisation verticale, pour effectuer une égalisation verticale de l'image fusionnée, l'égalisation verticale consistant à normaliser chaque sous-image de sorte que la luminosité et le contraste soient égalisés.

4. Dispositif d'acquisition d'image selon la revendication 3, **caractérisé en ce que** la valeur dudit chevauchement est de préférence de 5 cm à 7 cm.

5. Système radiographique, ledit système radiographique comprenant un tube à rayons X (1) et un détecteur (4) disposés sur des positions opposées, **caractérisé en ce que** ledit système radiographique comprend en outre un dispositif d'acquisition d'image selon la revendication 3 ou 4.

Fig. 1

Fig. 2

```
1) determination step

2) calculation step

3) capturation step

4) pasting step
```

Fig. 3

Fig. 4A

Fig. 4B

Fig. 4C

step 20)calculating a patient coverage on the plane of a region of interest based on the starting position and the ending position of the region of interest.

Step 21) calculating the number of the sub-images required to be captured based on the patient coverage, the distance from said detector incident plane to the plane of the region of interest, the distance from the focus to said detector incident plane and the value of said overlap

Step 22) calculating the component of said field of view at the direction of the tube movement based on the number of the sub-images required to be captured, the distance from said detector incident plane to the plane of the region of interest, the distance from the focus to said detector incident plane and the value of said overlap.

Step 23) calculating the positions of the tube and the detector corresponding to each sub-image based on the patient coverage, the component of said field of view at the direction of the tube movement, the distance from said detector incident plane to the plane of the region of interest, and the number of said sub-images.

## FIG. 5

40) cutting off the useless information in said sub-images

41) determining the search scope as required in the registering of the adjacent images based on the overlap value of the region of interest in the two adjacent sub-images

42) determining the relative positions matched between the adjacent images from calculating the similarities between the adjacent images based on said search scope

43) performing image merging on the corresponding pixels of the adjacent images based on said relative positions

44) conducting vertical equalization of the merged image.

## FIG. 6

Fig. 7

Fig. 8

Fig. 9

Fig. 10

Fig. 11

Fig. 12

Fig. 13

Fig. 14

**EP 2 250 965 B1**

**REFERENCES CITED IN THE DESCRIPTION**

**Patent documents cited in the description**

- EP 1484016 A
- US 20080152088 A

- US 7177455 B
- US 6944265 B